# EUROPEAN PATENT APPLICATION

(11) **EP 4 187 549 A2**
(43) Date of publication of application: **31.05.2023**
(21) Application number: 22210657.7
(22) Date of filing: 30.11.2022
(51) Int. Cl.: G16H 40/40, G16H 20/13, G16H 40/67, A61B 50/36, B65F 1/14

(54) **A SYSTEM, DEVICE AND METHOD FOR SUPPORTING MANAGEMENT OF MEDICATION USE, AND ADHERENCE TO MEDICATION SCHEDULES**

(30) Priority: 30.11.2021 GB 202117301
(71) Applicant: HealthBeacon PLC, D12 WD85 Dublin 12 (IE)
(72) Inventor: Shattock, Richard, Dublin 12, D12WD85 (IE); Joyce, James, Dublin 12, D12WD85 (IE); Daly, Kieran, Dublin 12, D12WD85 (IE)
(74) Representative: Murgitroyd & Company

(57) **Abstract**

The specification provides a point of care device for supporting management of medication at a point of care location the device comprising: a container having base, top and side walls defining an interior space accessible *via* an opening and a sensor system. The sensor system comprising: one or more vibration sensors, configured to detect vibrations or movements resulting from interactions with the container, wherein the interactions comprise end-user interactions to deposit used medication delivery devices into the container, and to output vibration signal data indicative of the vibrations detected; and a memory configured to store the detected vibration signal data over a defined time period and a power supply. The sensor system is configured for coupling to the container; the container configurable in a first point of care mode or a second processing mode, wherein in the first point of care mode the container is configured to receive used medication delivery devices into the interior space, and to contain said used medication delivery devices therein, and wherein in the second processing mode the container is configured to allow access to the interior thereof for removal of the used medication delivery devices.

## Description

### Field

The present application provides systems, devices and methods for supporting users in management of prescription medication use and adherence to medication schedules. The system is directed to providing support to the care provider and point of care users.

### Background Of The Invention

The present invention generally relates to medical adherence, and more specifically to supporting management of medication use and adherence including for end user patients at multiple points of care.

In general, medication adherence usually refers to whether patients take their medications as prescribed (e.g., twice daily), as well as whether they continue to take a prescribed medication. Medication non-adherence is a growing concern to clinicians, healthcare systems, and other stakeholders (e.g., payers) because of mounting evidence that it is prevalent and associated with adverse outcomes and higher costs of health care. Further, there are often particular issues relating to the use of medication that requires a specialised medication delivery device at an end user point of care - including safe collection and management of used medication delivery devices - which may include sharps, syringes or auto-injectors or other suitable devices. Therefore, there also exists a need for alternative or improved systems and methods that can support and assist individuals in maintaining medication schedules at point of care taking account of the above noted issues. A need also exists for an alternative or improved systems and methods to support care providers in management and care of point care end users and in management of medication schedules.

### Summary

These and other problems are addressed in accordance with the present invention by providing a method and system for supporting users in management of prescription medication use and adherence to medication schedules.

According to a first aspect, the present specification provides a point of care device for supporting management of medication at a point of care location, the device comprising:
a container having base, top and side walls defining an interior space accessible *via* an opening;
a sensor system comprising:
   one or more vibration or movement sensors, configured:
      to detect use events which comprise vibrations or movements resulting from interactions with the container, wherein the interactions comprise end-user interactions to deposit used medication delivery devices into the container, and
      to output vibration or movement signal data indicative of the vibrations or movement detected; and
   a memory configured to store the detected vibration signal data over a defined time period; and
   a power supply;
   the sensor system is configured for coupling to the container;
      the container configurable in a first point of care mode or a second processing mode, wherein in the first point of care mode the container is configured to receive used medication delivery devices into the interior space, and to contain said used medication delivery devices therein, and wherein in the second processing mode the container is configured to allow access to the interior thereof for removal of the used medication delivery devices; and
      wherein the sensor system comprises an accelerometer configured to detect vibrations or movement resulting from interactions with the container, in one or more directions (X, Y, Z), and to output a signal indicative of the movement or vibrations that have been detected.

This signal can then be analysed, including on the basis of calibration data and other related use data to determine the different kinds of use events, or user interactions there have been with the device, including time, date, frequency of the various types of use events.

In one arrangement the sensor system is a self-contained unit, wherein the memory comprises a local memory and the power supply comprises an exchangeable or rechargeable battery. In one arrangement the container is a sharps bin or sharps container. In one arrangement the used medication delivery devices comprise one or more of: syringes, injector or autoinjector devices, and/or sharps. In one aspect, the use events include one or more of deposit events, movement events such as moving, lifting or dropping the container, container location changes, opening and closing of the container opening, or container transport. In one aspect, the sensor system is configured for operation independently as a stand-alone device, the sensor system having an integrated memory and power supply, such that the sensor system does not require real-time connection to an external power supply or to an external communication network or connection to a power supply or external communication network during the time period that the device is located at a point of care site. The sensor system may be removably couplable to the container. The sensor system may be removably insertable into the container, and wherein the sensor system is removable for retrieval of vibration or movement signal data from the memory and/or for connection to a power supply interface for recharging of the power supply. The container may be configured for docking, while the sensor system is in place therein, to a communication interface, for retrieval of stored vibration signal data from the memory and/or to a power supply interface, for recharging of the power supply. The container may further comprise a receiver for receiving the sensor system and configured to provide secure location of the sensor system relative to the container. In one arrangement the sensor system comprises an accelerometer configured to detect vibrations or movement resulting from interactions with the container, and to measure the relative direction of the vibrations or movement in at least one axis, and to output a vibration signal indicative of the movement or vibrations that have been detected. The sensor system may comprise an accelerometer configured to detect vibrations or movement resulting from interactions with the container, in the x, y and z axes, and to output a signal indicative of the movement or vibrations that have been detected. The signal data is based on detection of vibrations or movements, resulting from end-user interactions with the container to deposit used medication delivery devices into the container provides data indicative of medication use by the end user at the point of care. The sensor system may be configured to detect and store the signal data arising from interactions with the container over a period of time, and wherein the sensor system is configured such that the data is retrievable or downloadable for analysis to allow determination of the dates and times of user interaction with the container, indicative of patterns of use of medication.

In another aspect, the accelerometer also detects and indicates in the signal output data any movement or acceleration experienced at the device.

According to another aspect of the specification there is provided a point of care medication management system for supporting and managing point of care medication care plans and for tracking medication care events associated with a point of care medication user, the system comprising:
an information system, comprising for each point of care user a point of care user record including a medication care plan for the point of care user;
a point of care container for collection of used medication devices, wherein the point of care container is deployed to the point of care for the point of care user for a predetermined period of time;
a point of care medication use event recordal system, configured to record medication use event data at the point of care relating to said period of time that the container is at the point of care, wherein the use event data is provided to the information system and to the point of care user record (215-1);
the system further comprising:
a returned container processing module, configured for receiving and processing returned containers and container contents, the container contents comprising the used medication delivery devices collected the point of care for the point of care user over the period of time;
wherein the processing of the contents of each returned container comprises:
   imaging the contents to obtain image data, and/or weighing the contents to obtain weight data;
wherein the image data and/or the weight data pertaining to the contents of the container returned from the point of care user is provided to the information system and to the relevant point of care user record, and wherein a point of care device (100) comprising a medication use event detection or recordal means (180) is provided at the point of care (POC-1) and the device is configured to detect or record use event data (270) and to communicate said use event data (270) to the information system.

In one arrangement the container includes an identifier which is read at the processing module to allow the image data and/or weight data relating to the contents of a container to be attributed to the relevant point of care user record.

In one arrangement, the container comprises a sensor system and the sensor system is configured to detect and store use event data and wherein the use event data is retrieved from the returned container at processing.

In another arrangement a point of care device comprising a medication use event detection or recordal means is provided at the point of care and the device is configured to detect or record use event data and to communicate said use event data to information system.

In one arrangement event data is communicated to the information system in real-time or at pre-determined intervals. The information system comprises an analyser which may be configured to analyse the use event data obtained over a time period in comparison with one or more of the following:
care plan for the point of care user;
image data; and/or
weight data;
wherein the image data and weight data relate to the used medication delivery devices collected at the point of care over the same time period.

The information system may be configured to determine whether the use event data is consistent with the image data and weight data and to indicate any inconsistency. The information system may be configured to determine whether or not the use event data is consistent with the user care plan for assessment of medication adherence and care plan adherence. The information system may be configured to analyse the use event data comprising data detected and stored by the sensor system to determine one or more of the times, dates, and frequency of use events. The information system may be configured to analyse use event data detected by an accelerometer to identify the occurrence of use events. The use event data may comprise movement and vibration signal data detected by the accelerometer including background signals and peaks signals, and the information system is configured to:
based on one or more of:
   (i) accelerometer calibration data,
   (ii) analysis of use event data, and one or more of related image data and/or weight data and/or care plan data;
   (iii) previous analysis of user event data;
   (iv) machine learning based on previous analysis of use event data,
   (v) machine learning based on previous analysis of user event data and related user medication care plans;
identify from the movement and vibration data, signals which are indicative of particular events, wherein the particular events include: use events indicating disposal of a used medication delivery devices into the container, change of location of the container, and transport of the container to and from the point of care, and the relevant times and dates.

The information system may comprise an interface configured to allow a care provider to enter a point of care user care plan for a point of care user or to access data relating to use of medication by a point of care user including adherence.

The information system may comprise a neural network configured to receive as inputs one or more of a medication care plan, use data, image data, weight data, the data relating to a point of care user record and to a common sample time period and to process the data.

The system may further comprise an in-line processing system for processing of returned containers and container contents, the processing of the containers comprises one or more of the following:
- processing of the contents of returned containers to recover and to recycle used medication delivery device components and to process waste.

The image data, obtained by imaging the contents of the container, may be analysed by the system.

The system may be configured to analyse the image data to identify the type of medication delivery device to provide one or more of:
(i) classification of medication delivery devices by type;
(ii) sorting of medication delivery devices by type for recycling or waste processing;
(iii) grouping of medication delivery devices by type for batch recycling or waste processing;
(iv) identification of contents to provide information about use of medication at the point of care.

According to a still further aspect of the teaching of the specification there is provided an information system for management and support of point of care medication programmes, the information system comprising a data module configured to receive and store data, an analyser module configured to analyse the stored data
wherein the data module is configured to receive:
   care plan for the point of care user, wherein the care plan is received at an interface;
wherein the data module is further configured to receive data relating to use of medication by a point of care user at a remote point of care, the data relating to use comprising:
   use event data as detected by a sensor system located at the point of care for a sample time period;
   and one or more of the following:
      image data as detected by an imaging device configured to image the contents of a used device collection container when returned from the point of care location, the container containing used medication delivery devices collected over the sample time period;
      weight data as detected by a weighing device configured to weigh the contents of a used device collection container when returned from the point of care location, the container containing used medication delivery devices collected over the sample time period;
   wherein the image data and weight data relates to the used medication delivery devices collected at the point of care over the same sample time period;
   wherein the use event data, image data and/or weight data are attributed to the point of care user record for the relevant point of care user in the data module.

The information system may be configured to determine whether the use event data is consistent with the image data and weight data and to indicate any inconsistency.

According to a further aspect of the specification, there is provided a sensor system comprising an accelerometer configured for coupling to a sharps bin and configured to detect and store vibration signal data indicative of user interaction with the sharps bin to which the sensor system is coupled.

In one arrangement the sensor system is calibrated for use in a sharps bin, to detect user interactions with the sharps bin to deposit a used medication device into the bin. The sensor system is configured to detect and store such interactions as use event data. The sensor system is configured to allow downloading or retrieval of the signal data. The signal data comprising vibration signal data from the accelerometer. Taking account of calibration of the sensor system for detection of typical user interactions, the vibration signal data is analysed to allow identification of different use events as detected by the sensor system. The vibration signal data provides information relating to end user use of medication. The sensor system may in one arrangement be placed in the base of the sharps bin. The sensor system may be another arrangement be mounted on a platform in the sharps bin. The sensor system may advantageously be provided as a self-contained unit for use with a sharps bin.

### Brief Description Of The Drawings

Figures 1 and 2 show exemplified implementations of a system 1000 for supporting the users including care providers in providing and managing medication for end users at multiple points of care, and in monitoring and supporting medication adherence in accordance with embodiments of the present specification;
Figures 3A, 3B are schematic illustrations of an exemplified container device according to embodiments of the present specification;
Figure 4 is a schematic of an exemplary sensor system according to arrangements of the present specification;
Figure 5 shows a process flow of an exemplary method 610 for providing a container device to a point of care user and use of the container device to sense use data in accordance with embodiments of the present specification;
Figure 6 shows a process flow of an exemplary method 630 for capturing and processing use data in accordance with embodiments of the present specification;
Figures 7 and 8: show examples of a sample of vibration signal data 220 demonstrating the events relating to disposal of a medication delivery device 500 into the device 110. The data is sensed using a sensor system 150 according to an exemplary arrangement of the specification. The event vibration signals 225 are indicated for X, Y and Z directions - and the data shows Vibration peaks 216 against a background 226 signal. The vibrations in X, Y and Z are plotted against time - the graph shows data from a sample period of time.
Figure 9 shows exemplified implementations of system 1000 for supporting the users, including care providers in providing and managing medication for end users at multiple points of care, and in monitoring and supporting medication adherence in accordance with embodiments of the present invention - this arrangement includes communication of use data from the remote POCs to the system 300, 350 - as in the examples of Figures 1 and 2, the use data is linked to the user record and also to the image and weight data pertaining to the contents of the container when returned from the POC;
Figure 10 shows a process flow of an exemplary method 650 for capturing and processing use data in accordance with embodiments of the present specification;
Figure 11 shows a block diagram illustrating an exemplary arrangement for capture/input/communication of different data items - use/image/weight and an exemplary arrangement of an information system in accordance with embodiments of the present specification;
Figure 12 shows a block diagram illustrating flow 670 of data inputs to the system 350 - use/image/weight and an exemplary arrangement of an information system in accordance with embodiments of the present specification;
Figure 13 shows a block diagram illustrating flow 680 of data inputs to the system 350 -in connection with the container record and container management;
Figure 14 shows a block diagram illustrating an in-line process/system for processing of container and container contents in an exemplary arrangement in accordance with embodiments of the present invention in the arrangement of Fig 14 - the processing is moved through a number of stages in a continuous in-line process;
Figure 15 shows a flow diagram illustrating data and contents processing flow according to an exemplary arrangement of the present invention
Figures 16A, 16B, 16C and 16D are schematic illustrations of an exemplified container device according to embodiments of the present invention, similar to those of Figs 3 and 4.

### Detailed Description Of The Drawings

The present invention will now be exemplified with reference to the accompanying drawings.

Throughout the following discussion, numerous references will be made regarding servers, services, interfaces, portals, platforms, or other systems formed from computing devices. It should be appreciated that the use of such terms is deemed to represent one or more computing devices having at least one processor configured to execute software instructions stored on a computer readable medium. For example, a server can include one or more computers operating as a web server, database server, or other type of computer server in a manner to fulfil described roles, responsibilities, or functions. One should further appreciate the disclosed computer-based algorithms, processes, methods, or other types of instruction sets can be embodied as a computer program product comprising computer readable media storing instructions that cause a processor to execute the disclosed steps. One should appreciate that the systems and methods described herein involve interconnected networks of hardware devices configured to receive data using receivers, transmit data using transmitters, and transform electronic data signals.

The following discussion provides many exemplary embodiments of the inventive subject matter. Although each embodiment represents a single combination of inventive elements, the inventive subject matter is considered to include all possible combinations of the disclosed elements. Thus, if one embodiment comprises elements A, B, and C, and a second embodiment comprises elements B and D, then the inventive subject matter is also considered to include other remaining combinations of A, B, C, or D, even if not explicitly disclosed.

For simplicity and clarity of illustration, reference numerals may be repeated among the figures to indicate corresponding or analogous elements. Numerous details are set forth to provide an understanding of the examples described herein. The examples may be practiced without these details. In other instances, well-known methods, procedures, and components are not described in detail to avoid obscuring the examples described. The description is not to be considered as limited to the scope of the examples described herein.

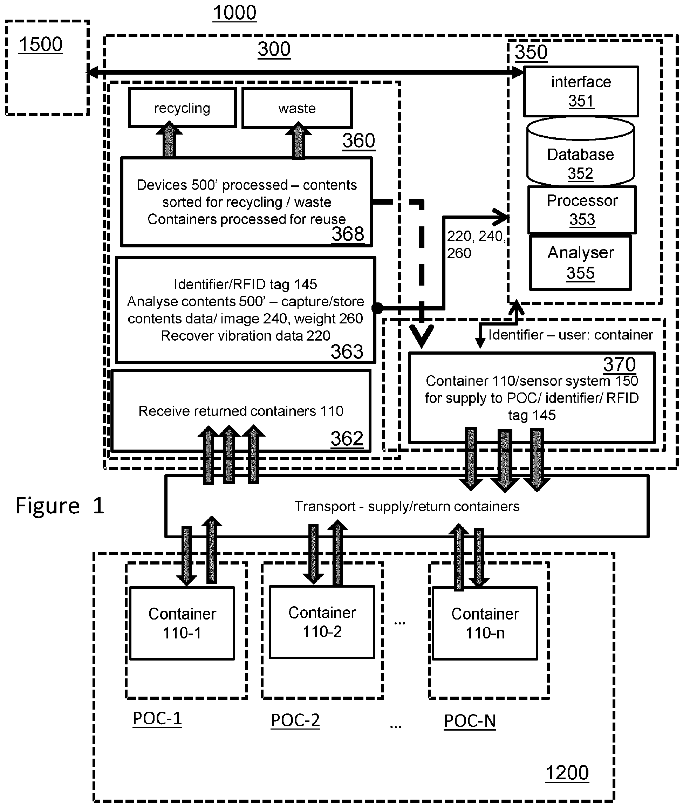

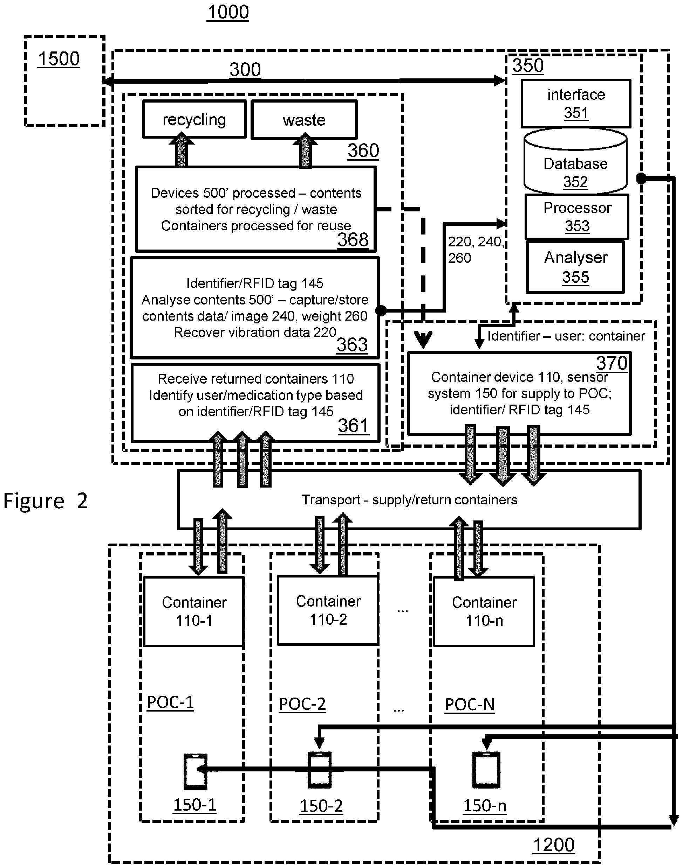

Referring to the Figures, and initially in particular to Figures 1 and 2, which are flow diagrams illustrating in overview two possible arrangements of an exemplary system 1000, according to the specification for supporting management of medication use at point of care, as described. The main entities and activity centres in the overall system are the care providers, CP, 1500 (CP-1, CP-2..., CP-N), the support provider 300 and the point of care, POC, users 1200 (POC-1, POC-2... POC-N). Fig 1 shows in overview exemplary entities and flows of data, and the management of supply and return of physical devices between the various entities. System 1000, methods 600, support provider systems 300 and device 110 are aimed at providing improved support for care providers and for patient users at point of care, POC, including to support the management and use of medication and adherence to medication schedules. The systems and methods further provide for management of used medication delivery devices including syringes or autoinjectors, and for the processing of used medication delivery devices 500 at the support provider 300.

The system 1000 of the present specification is configured to provide and support communication feedback to the care provider and to the patient end user in management of medication, and in following or adhering to a prescribed schedule for use of medication. The management of data and the various data inputs and outputs within the system, and of physical devices including containers 110 and used medication delivery devices 500', is handled in the exemplary arrangements centrally by and *via* the support provider 300 entity or hub.

The present specification also provides a method 600 for management of medication care plans, of medication delivery devices 500, and used medication delivery devices 500' and for monitoring of use of the medication to support adherence to a medication schedule. The system 1000 and method 600 herein include use of an end-user point of care device 110, also described in further detail below.

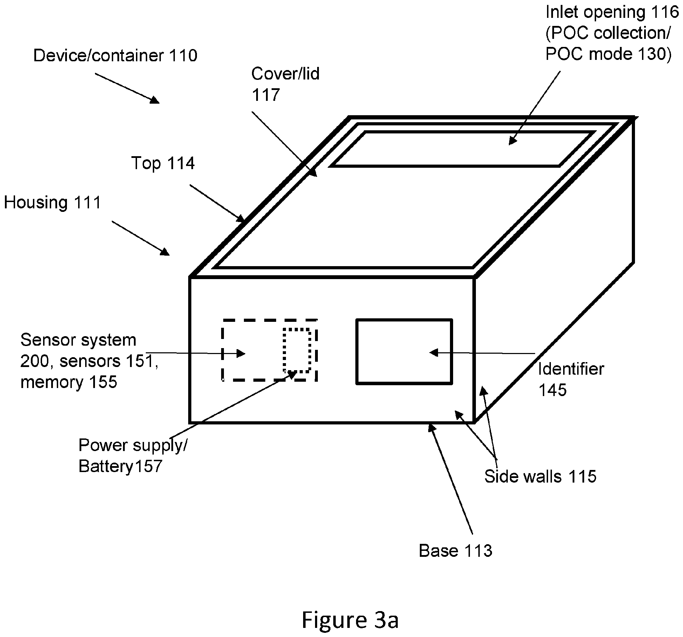

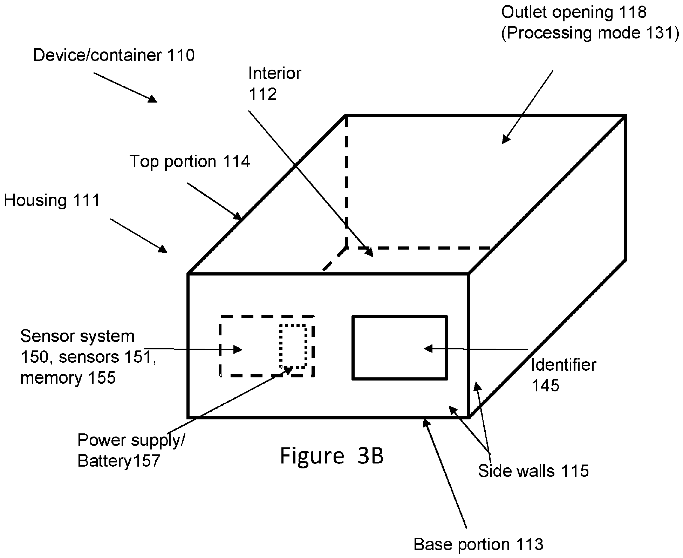

Referring to Figure 3, an exemplary end-user point of care device, a container 110 comprising a sensor system 150, is described.

The sensor system 150 is configured to detect movement or vibration arising from interactions with or activity at the container 110 over a sample time period and to output, and to store the detected vibration signals 220 in a local memory.

The relevant sample time period may be from when the container leaves/is collected from the support provider 300 for transport to the point of care to the time that it is retuned after use. The relevant period of time may be from when the container is deployed at the point of care to the time that it is collected from the point of care. The period of time is typically 2 or 3 months. It will be appreciated that different periods of time may be selected, as required.

In the exemplary arrangement of Figures 3a and 3b, container 110 comprises a container housing 111 having a base 113, top 114, and side walls 115 defining an interior space 112. The container 110 further comprises an inlet opening 116 which allows items to be inserted into the interior 112 of the container. The container is normally circulated to users as a closed container (with cover 117 in place), in a first point or care mode 130. The container is configured to receive and collect used medication delivery devices 500'. The inlet opening 116 may be shaped and formed to allow insertion of specified type devices only. The container 110 may be configured to be opened to allow access to the interior 112 and the items therein, used medication delivery devices 500' at the support provider 300 for processing, when the container is in a second processing mode 131 (the cover 117 is removed). The container 110 may comprise an outlet opening 118 that can be opened to allow access to the interior 111 of the container for emptying, cleaning and to allow access to the sensor system 150 and memory 155 for downloading of the stored data 220. The outlet and inlet openings 116, 118 may be at the same location on the container. The container 110 may comprise a cover 117 or lid. The cover 117 may in a first position define the inlet opening 116 and when removed, define the outlet opening 118.

### Sensor system

The sensor system 150 comprises one or more vibration or movement detection sensors 151 configured to detect vibrations arising from user interactions with or activity at the container 110 and to output a vibration signal 220. The sensor system 150 further comprises a memory 155 configured to store vibration signal data 220. The sensor system 150 further comprises a controller or processor 156. The sensor system further comprises a power supply or battery 157.

The one or more vibration detection sensors 151 may include for example one or more of the following: an accelerometer 152, a piezoelectric based sensor 153, or other suitable movement or vibration detection sensor 154.

In the exemplary arrangements of Figures 3a and 3b, the sensor system 150 comprises an accelerometer 152 configured to detect and store to the memory data 220 relating to movement, displacement or vibration in three directions, i.e. in the directions of axes X, Y and Z arising from interactions with the container.

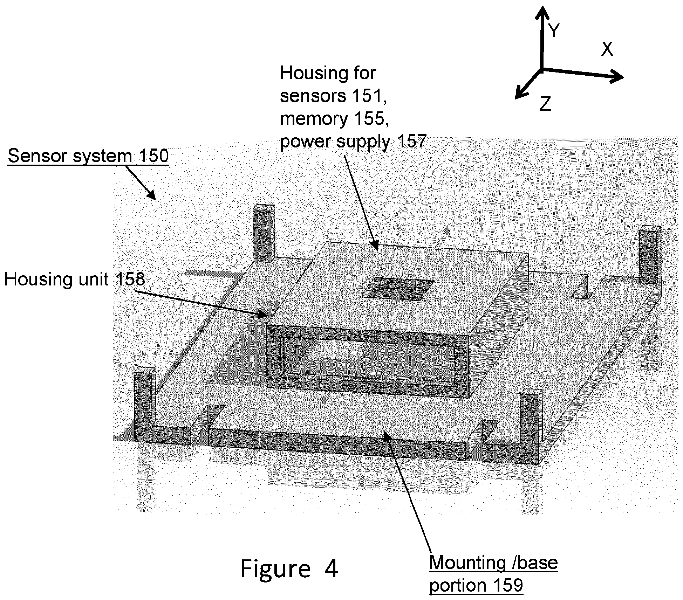

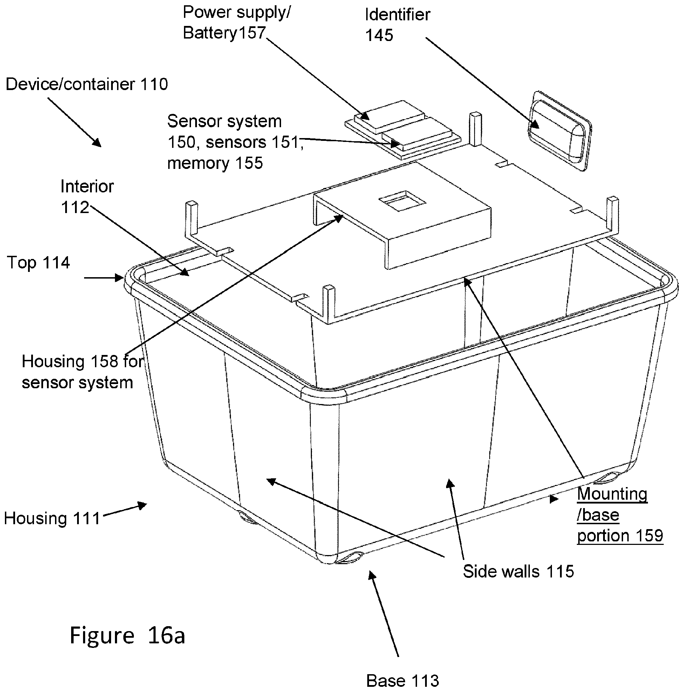

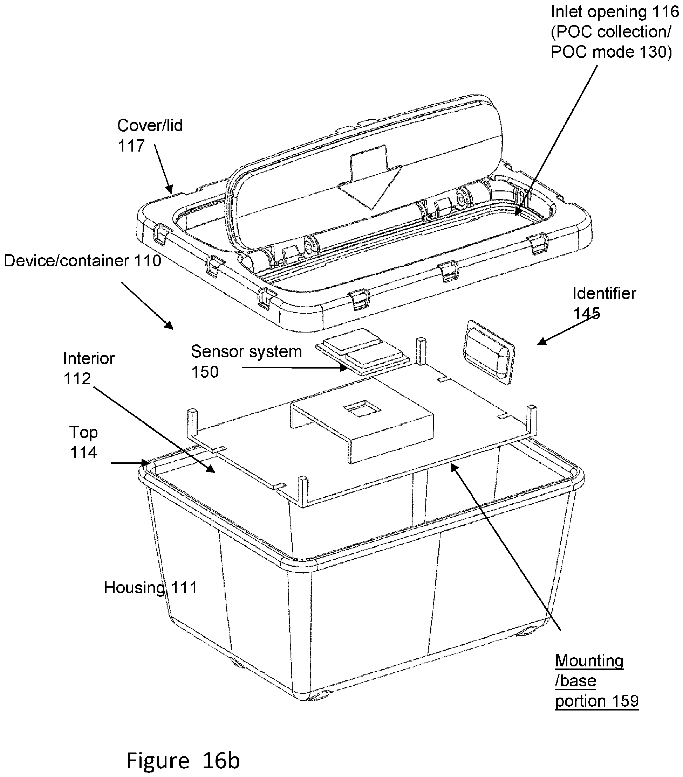

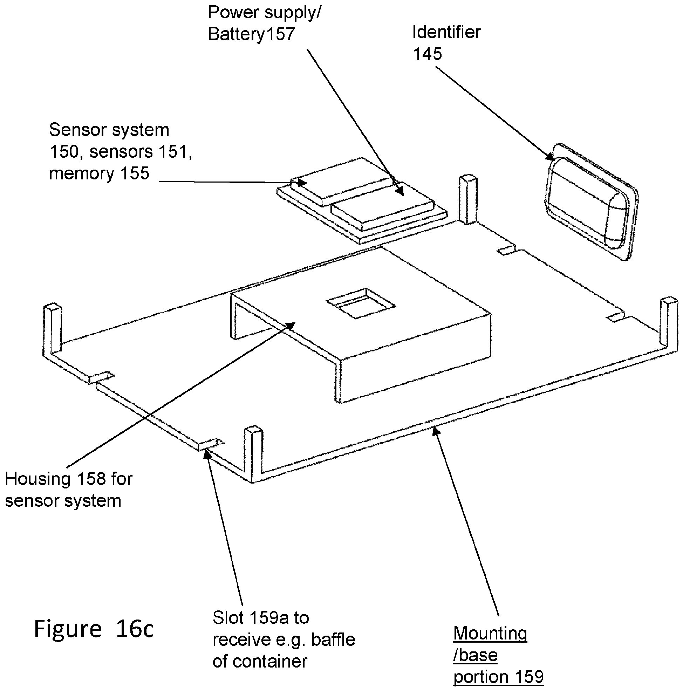

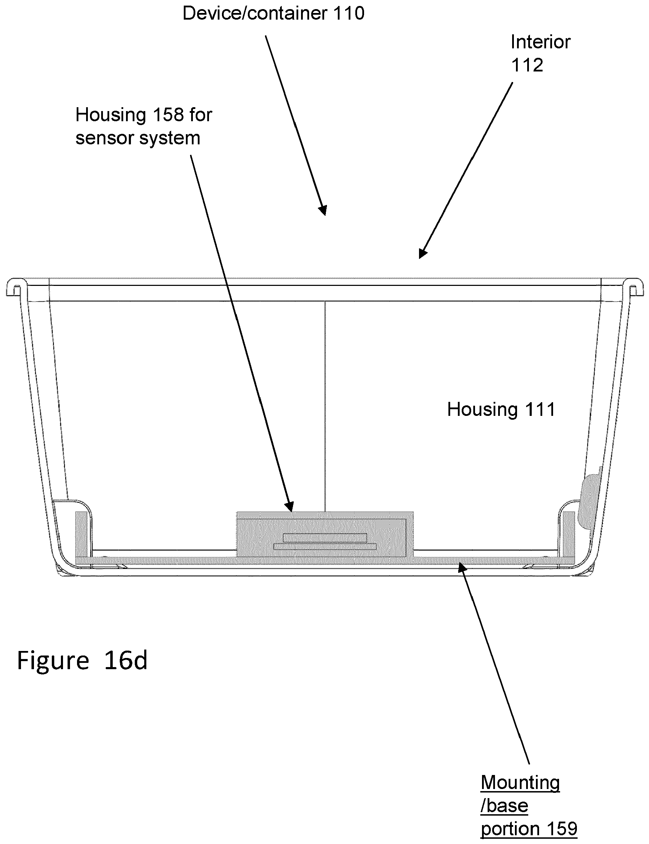

Referring to Figures 3a, 3b and 4 in the exemplary arrangements, the sensor system 150 may be mountable to or receivable in a sensor housing unit 158. The one or more sensors 151 and memory 155 may be located in the housing unit. The battery or power supply 157 may also be included in the housing unit. The sensor housing unit 158 comprises a base portion 159 formed for connecting or supporting the sensor system 150 in the container or supporting the sensor system in the base of the container. With reference to Figures 16a to 16d, further arrangements of a container 110 having a sensor system 150 mountable to a sensor housing 158 are shown. In the arrangements of Figure 16, as in Figure 4, a mounting base 159 is provided. The mounting base 159 is sized to be received in the base of the interior of the container 110. In particular, the mounting base 159 is configured to fit and maintain its position and the position of the sensor system 150 mounted therein, relative to the container during use. The sizing of the mounting base 159 is such that movement in x and/or y and/or Z relative to the container 110 is prevented. In a further arrangement the container may include baffles or other features and the mounting base includes receivers or slots 159a to receive the baffles or features. It will be appreciated that other suitable alternative corresponding features may be provided - on the container 110 and mounting portion 159. With the arrangement of Figure 16 the sensor is located at the same position throughout the period of time that the container and sensor are deployed and as the base fits the container there is no movement that might affect the sensor data.

The housing unit 158 may comprise a cradle to hold the sensor system 150. The housing unit 158 may further comprise an additional compartment to house a secondary battery 157' useable with the sensor system to extend the detection lifespan. The housing unit 158 may be sealable to avoid ingress of liquid or contaminants. The sensor system 150 may be mounted using a resilient mounting for example a spring mounting. The skilled person will appreciate that other suitable mounting arrangements may also be provided.

The memory 155 may be integrated with the sensor system 150 or may be connectable thereto. The power supply or battery 157 may be removably insertable into the sensor system 150 or may be connectable thereto.

In a further arrangement a solar panel may be provided to charge the battery 157 or power supply. The solar panel may be removably attachable to the container 110. The solar panel may be integrated into a container wall. The solar panel may be located on a side wall or the cover of the container.

The container 110 further comprises an identifier 145. The identifier 145 is a unique identifier to identify a particular container 110 and to allow that the container 110 is attributable to a particular end user e.g. user at POC-1, at a point of care site and to the patient care record 215 e.g. 215-1. The identifier 145 may comprise a RFID tag. In a further arrangement RFID tagged sensors may be provided for tracking and identifying the individual containers. When the container is prepared for dispatch to a POC user the identifier 145, e.g. 145-1 is linked to that user record 215-1 and dispatched to that user at the particular POC for the relevant time period.

Based on the identifier 145, the data 220 stored in the memory of a container 110 during the time period that the container was located at the point of care, can be linked to the relevant end user. Similarly, data 240 or 260 relating to the used medication delivery devices 500' collected in the container 110 over the sample time period that the container is in use can be linked to the relevant end user by the identifier 145.

In an alternative arrangement the container 110 may further comprise a receiver 120 for receiving the sensor system 150 or sensor system housing 158 and locating securely in a fixed position relative to the container body. The form and shape of the receiver 120 is configured to conform to that of the sensor system housing 158. The receiver could be positioned on the base or side walls of the container for example, being moulded as part of the manufacturing process. Alternatively, the receiver could be housed in the upper lid section. Access to the compartment could be gained by opening and closing a hinged or clipped door that secures and encloses the sensor system. It will be appreciated that various suitable receivers 120 or mounting arrangements may be used to locate the sensor in the container 110 or to connect it thereto. Some exemplary arrangements are described below. In a still further exemplary arrangement the receiver 120 is configured to mate with a corresponding connector on the sensor system 150. The container and sensor system having corresponding features to allow their interconnection. The receiver 120 and sensor system are configured such that when the sensor system is located in the receiver or connected to the receiver, the sensor is located in a single fixed position relative to the container. The arrangement limits or prevents movement of the sensor system 150 relative to the container. The sensor system 150 is accordingly correctly position for detection of vibrations or movement, resulting from interaction at the container. In one arrangement, the container 110 and the receiver 120 may comprise a compartment 121 configured to receive the sensor system 150 including sensor 151, memory 155 and power supply 157. The compartment 121 may comprise a seal 122 to prevent ingress of liquids. Effectively in this arrangement the sensor system housing is defined by the compartment 121 integrally formed in the container 110. The form and shape of the compartment 121 is configured to conform to that of the sensor system, memory and power supply to effectively locate and secure the components within the container. The arrangement limits or prevents movement of the sensor relative to the container when the sensor in deployed to actively detect vibrations arising from interactions or activity at the container. In a further arrangement, the sensor system 150 may be inserted into the interior 112 body of the container 110 as the cover is applied and the container prepared for deployment. The sensor system in this case simply rests on the base 113 of the container. In a further arrangement the sensor system 150 may comprise or include a locater element 165 that is locatable between the container cover 117 and container side wall 115 when the cover 117 is attached to the container, as it is prepared for distribution to a user at a point of care. The locater element may for example be a hook that is hooked over the side wall and secured in place between the side wall and cover to locate the sensor system in the interior of the container.

In another arrangement, the receiver for the sensor system 150 may comprise a moulding in the container lid or cover 117 or housing 111 to house the sensor system 150. In a further arrangement, the receiver 121 may comprise a moulding in the container body to house the sensor system 150.

The sensor system 150 or sensor system housing 158 may be removably receivable in the interior 112 of the container 110. According to the various arrangements, including as noted above. However, it will be appreciated that the compartment or receiver may be located on the exterior or interior of the container 110 using one of the receiver or connection or attachment arrangements described above. Similar suitable arrangements may also be used. The sensor system 150, or the sensor housing unit 158 and/or memory 155 may be removable for docking to a docking station to allow retrieval of data 220. The sensor system 150 may further be removable for docking for rechanging of battery 157. In an alternative arrangement, the container 110 comprising the sensor system 150 and memory may be docked to a docking station for retrieval of data 220 from memory 155 and/or recharging of the battery 157, without the need to remove the sensor system from the container 110. In this arrangement, the container 110 may comprise one or more ports formed in a wall of the container to provide access to the sensor system or memory or battery. In an alternative arrangement, access to the sensor system and/or the memory and/or battery may be via an external access panel. In a still further arrangement the communication may be wireless e.g. Bluetooth or other NFC system

The sensor system housing 158 or the container 110 may comprise ports to allow access to the memory 155 or battery 157 contained in the respective locations.

As noted above, Figures 1 and 2 provide an overview of the entities of the system 1000 of the specification, including some of the data management and processing flows and the management at the support provider side 300 of the devices 110 and various data inputs 220, 240, 260, 270, 290 and outputs.

The key entity of the methods 600 and system 1000 of the specification is the support system 300 or support provider system 300. The support system 300 comprises an information system 350. The information system 350 a computing system including memory and processor. The system 350 comprises a database module 352, processor module 353, and analyser module 355. The system also includes a care provider interface 351 configured to allow a care provider (CP-1, CP-2...CP-N) enter a care plan 310 for POC end users (POC-1, POC-2 .. POC-N) and to access data relevant to the POC end users who are patients of that care provider. The information system 350 is configured for data management of the user data and use data and data pertaining. The information system 350 also provides for management of data pertaining to the processing of containers and to the processing of container contents 500'.

The system 300 includes a container deployment module 370 where containers 110 are prepared for supply to POC users.

The system 300 includes a returned container processing module 360, where returned containers 110 are received and where the containers 110, data 145, 220, 240, 260, 290 and container contents 500' are processed.

The processing module includes a first module 362, the receiver module, configured to receive containers. At the receiver module 362, the container identifier 145 may be scanned or read to identify the container and from that the relevant user record. From the user record it is possible to identify the medication type and accordingly the expected contents - i.e. type and number of used delivery devices 500' contained in that container (medication type being associated with the user record 215 and the identifier 145). Containers 110 may accordingly be pre-sorted or pre-classified for further processing including based on medication type - this allows for processing of similar used medication delivery devices (500') in batch.

From the first receiver module 362, containers 110 are provided to a second module 363.

In the second stage of processing at module 363, the contents are recovered from a container 110 and the data 220 is recovered. The contents may include the sensor system150 - or battery 157 or memory 156 and the used medication delivery devices 500'.

Processing includes:
- Reading the identifier 145. The identifier will be/is used to allow the vibration data 220 (use event data) and contents data 240, 260 from the container or associated with the container and to be attributed to the relevant POC user record 215.
- Opening the container/removal of the lid 117. The lid 117 may be recyclable or reusable and is processed accordingly.
- Recovery of the stored vibration data 220. The data 220 comprises use event data. The vibration data 220 is sensed by sensor system 150 and stored in a local memory 156 in the container 110 for a sample time period for example during which the container is at the POC site POC-1 or for a sample time period between dispatch of the container and return to the support provider 300.

Data 220 may be recovered by removing the memory 156 or sensor system 150 from the container 110 for docking or connecting to allow recovery of the data 220. Alternatively, the data may be recovered wirelessly. Alternatively, the data 220 may be recovered by docking the container 110 and integrated memory to a docking cradle. The power supply 157 may also be recharged by docking the container to a power supply. As described above, the power supply may be charged using a solar panel attachable to the container. The data recovery and recharging will depend of the arrangement of the sensor system used.

### - Recovery of contents - Analysis of contents - Capture and store data relating to the contents.

The containers 110 upon return are expected to contain used medication delivery devices 500' as deposited therein during the time period that the container was located at the POC side. When a container 110 is opened and emptied the contents 500' are weighed and imaged. The module 363 includes an imaging system or machine vision system 364 and weighing device 365. As noted above, the module 363 may also include a power supply 366 for recharging and data communication means 367. The image data 240 and weight data 260 are provided along with vibration data 220 and the identifier 145 to the information system 350. The POC use data - including image data 240, vibration data 220, weight data 260 are attributed to the relevant POC user data records. The use data can therein be analysed also in comparison with the relevant care plan 210. The data set 216 for a user for a sample time period that the container is at a point of care) (220 (and/or 270), 240, 260) can also be analysed in comparison with previous data sets 216 for the same user for a previous sample time period.

The image data 240 allows for identification of the used medication delivery devices 500' received in a container - includes number and types - and for identification of any foreign objects. The weight data 260 provides an indication of the number of items/and is comparable with the image data and care plan data. The expected weight for a particular number of devices 500' of a particular type will be known. The expected number of devices, weight, type can also be compared with the care plan.
- In further stages at module 368 the contents 500' (used medication delivery devices 500') are processed and the containers 110 are processed.

The activity includes the sorting of contents 500' for recycling, or for the recovery of reusable components. In addition, materials or components that cannot be recycled are processed for waste. The activity may further include the dismantling of the components of the medication delivery devices 500'.

The outputs from the processing of the used medication delivery devices 500' i.e. the components and materials (from the used medication delivery devices 500') may also be further analysed during processing - e,g, by imaging or weighing at intervals- to allow data collection and for reconciliation of the inputs and outputs for waste management by recycling and for disposal.

The image data comprises a second data set indicative of end user use of medication at the point of care. Essentially, this the image data is a second independent data set in addition to the detected use data. Both the image data and the use data can be considered in analysis of use of medication at the point of care. The image data and use data can be verified against each other.

Similarly, the weight data (relating to the weight of the contents) provides further feedback relating to use of medication on site.

The image data can also be used to classify and sort the returned containers and content for processing in batch. The image data may be user to sort containers or contents. The image data and/or weight data may be used to identify any contaminants.

Further, the containers 110 are processed for re-use. This includes cleaning and/or sterilising and/or inspection. The preparation for re-use may also include the recharging of the power supply and resetting of the sensor system. Some parts of the container may be recycled for example the cover or lid 117.

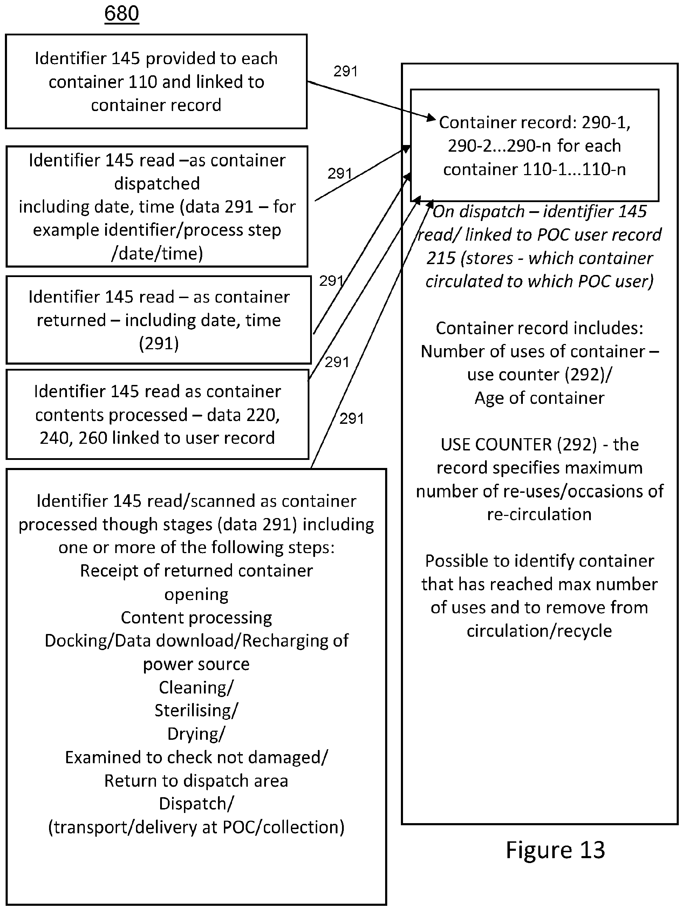

The container identifier may be scanned or read at one or more of the various steps of processing of the container and container specific data 291 provided (based on the scan/identification of the container at the various stages of processing though the system 360 and 370) to a container record 290. For example the identifier may be read when he container is returned and data 291 (including for example date and time of return and identifier) may be noted to the relevant container record 290. As noted previously the identifier is read on return also to allow linking of the container contents/use data to a user record 215. Referring also to Fig. 13 data 291 may be captured at various steps of the container processing.

In an exemplary arrangement of the system and method, during the waste processing and removal, the lid 117 is removed from the container 110 automatically, and as the lid is removed, a docking system in the module 363 engages the sensor system 150 and downloads the data 220. The contents 500' are tipped into a tray for image capture, the image data 240 is paired or associated with the sensor data 220 for analysis and validation also with the prescribed medication schedule of the care plan 210.

The output from the processing of the returned containers 110 content 500' therefore includes:
- data - user data 220 (and or user data 270), image 240, weight 260, identifier 145;
- data pertaining to container processing - data 291 - container identifier scanned at selected check points - e.g. when washed - scanned - data 291 captured (data 291 includes one or more of scan station e.g. wash and time, date, identifier -provided to container record 290). Allows verification that container has been processed through all steps.
- the used medication delivery devices 500' - image 240 of container contents/ weight 260 of container contents for a particular container/user- also of the contents as sorted into components and waste - may be imaged and/or weighed at intervals;
- the container/sensor system/power supply for reuse.

The support provider system 300 comprises a care provider interface 315 to allow care providers (CP-1 - CP-N) 1500 to enter care plan 210 including point of care user information 215 and details of the care plan medication information 216 including medication type, schedule etc. The care plan 210 is stored in information system 350.

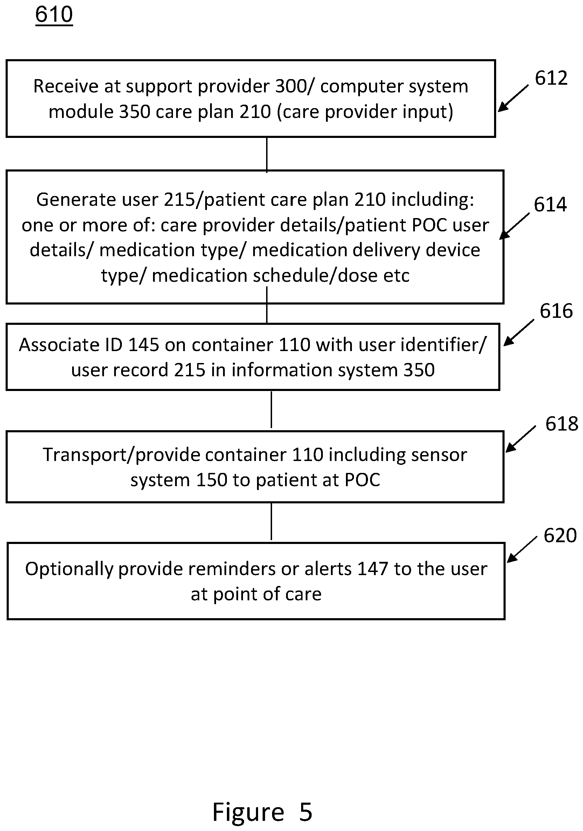

Referring to Figures 1 and 2 and to Figure 5, the method 610 is described. In an exemplary method 610 of Figure 5 when a care plan 210 is received, a user record 215 is generated that can be associated with a container for a particular circulation of the container to the user at the POC (for preparing a container 110 for supply to the user) by for example linking the user record 215 to an identifier 145 on the container. The container identifier 145 may comprise a RFID tag (attributable to the user / user record). It will be appreciated that other suitable identifiers may be used.

The method 610 relates to the set-up of a care plan 210 for a point of care end user 215 and preparation of the container 110 for deployment to the POC.

The method includes:
- receiving 612 at the support provider 300 computer system 350 a care plan 210-1 for a point of care user POC-1;
- Generating 614 point of care user POC-1 record 215-1 in information system 350 including care plan 210-1 including one or more of: care provider details CP-1, point of care user details POC-1, medication type, medication delivery device system/medication schedule/dose etc. The record will also include identification of container type appropriate to the relevant care plan and indicate the time period that the container is to be on -site at the point of care. Containers of different volume may be provided depending on the anticipated volume of use of medication and medication delivery devices. The record will also include any scheduling and logistical information for providing and collecting a container and for replacing the container.
- Associate 616 user identifier/ user record 215 with identifier 45 on the container 110. This allows for a particular container (and data 220, 240, 260 from the sensor/contents analysis) to be attributed to the relevant end user POC-1 data record 215-1;
- Provide 618 container 110 to the end user patient at the point of care POC-1;
- The method may further optionally include for providing reminders 147 or alerts to the end user at the point of care POC-1 - for example via sms to their mobile phone. Another communication system or means may also be used if preferred by the patient. Reminders are generated based on the care plan 210 and point of care user record 215.

Referring to Figures 1 and 2 and to Figure 5, the method 630 is described. The method 630 relates the inputs and flows of data relating to the use of medication delivery devices at the point of care.

The method 630 includes:
- Sensing 631 of movement or vibrations indicative of user interaction with the container 110. The data is detected over a sample period of time while the container is at the point of care. The data provides an indication of use of a medication delivery device based on the detection of disposal of used device 500 into the container.
- Storing 632 vibration/movement signal data 220 in memory 155 over sample time period;
- Return 634 of the container to the support provider 300;
- Retrieval and capture of data 636 relating to use of the container and the contents of the returned container. Data 220 is downloaded from the container, the contents are image to provide image data 240, the contents are weighed to provide weight data 260; the container identifier 145 is read to allow association of the use data 220 and contents data 240, 260 with the relevant user record 215;
- Providing 638 data 145, 220, 240, 260 to the information system 350 for association with the patient record 215 and care plan 210;
- Analysis 640 of use data including one or more of vibration movement signal data 220, image data 240 (contents), weight data 260 (weight of contents). Use data may be compared with data relating to the contents. Use and contents data may be compared with the care plan 210 including the expected schedule of use over the sample time period, and the expected contents at the end of the sample time period.

The image data 240 may include for example information identifying the number and type of medication delivery devices 500' collected in the container 110 at the POC. Device serial numbers relating to devices that have been placed into the container at the point of care during the relevant time period may also be obtained from the image data.

The weight data 260 includes the weight of the overall contents of the device. The recorded image data 240, weight data 260, and use data 220 are associated with the identifier 145 and the relevant user record 215 and care plan 210. The data 220, 240, 260 can be analysed together with the other data also to see if the measured data is in accordance with the expected use data for a care plan. Analysis of the data 220,240, 260 in comparison also with the care plan 210 provides insights into adherence to medication regimes.

A verification procedure may be performed that reconciles the vibration sensor data 220 including the date and time at which vibrations indicative of use of a medication delivery device occurred, with the prescribed schedule of the care plan 210 to compare the captured use data 220 and expected use data (based on the care plan 210). The use data is further validated by comparison of the data 220 with what was actually in the container i.e. the contents when it was returned, taking account of the image data 240. Clearly the contents data (240, 260) and the use data (220) all relate to the same activities placement of used medication delivery devices into the container 110 over the time period that the container is on location at the point of care

At the point of the care, the container 110 performs various functions including first of all, that of securely collecting and containing used medication delivery devices 500' for return and processing. The container 110 also provides monitoring at the point of care of interactions with the container, which is detected and stored as data 220 over the relevant time period, the data 220 relates to and is indicative of use of the container and of the medication at the point of care.

As described above, the sensor system 150 is configured to detect activity, interaction, or events at the container 110 by the detection of vibrations or movement using one or more sensors 151 and to output vibration signal data 220, which is stored in memory 155.

Vibration signal data 220 is collected over a pre-determined time period. Vibration signal data 220 may be collected and stored over the entire time period that the container is located at the point or care. Alternatively, the data may be collected and stored over a sample period of time less than the time period on site. Vibration signal data may also be collected and stored between the point in time from when the container is processed and configured for transporting to the point of care, when in location at the point of care and including exchange and return of the container to the-supplier side for processing. The time period may for example be 3 months. This allows for the regular collection and processing of used devices 500' and data 220, even if the container 110 has not reach capacity. The time period may be set in view of the medication regime and taking account of the number of medication delivery devices 500 expected to be used in a period of time and when the container 110 is expected to be nearly full or nearing capacity. Alternatively, the container size i.e. volume may be selected to support a particular selected time period and taking account of the medication regime.

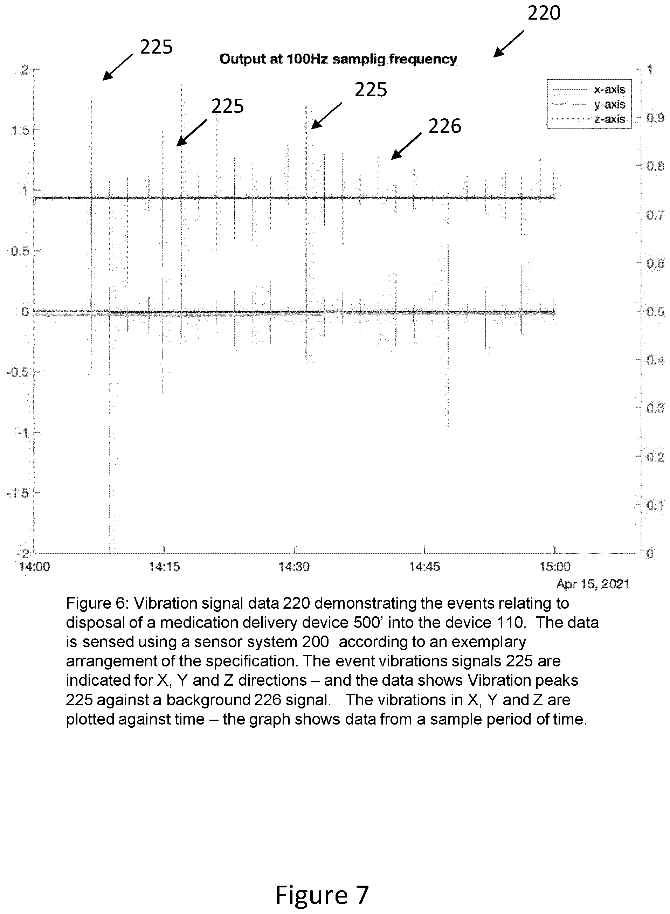

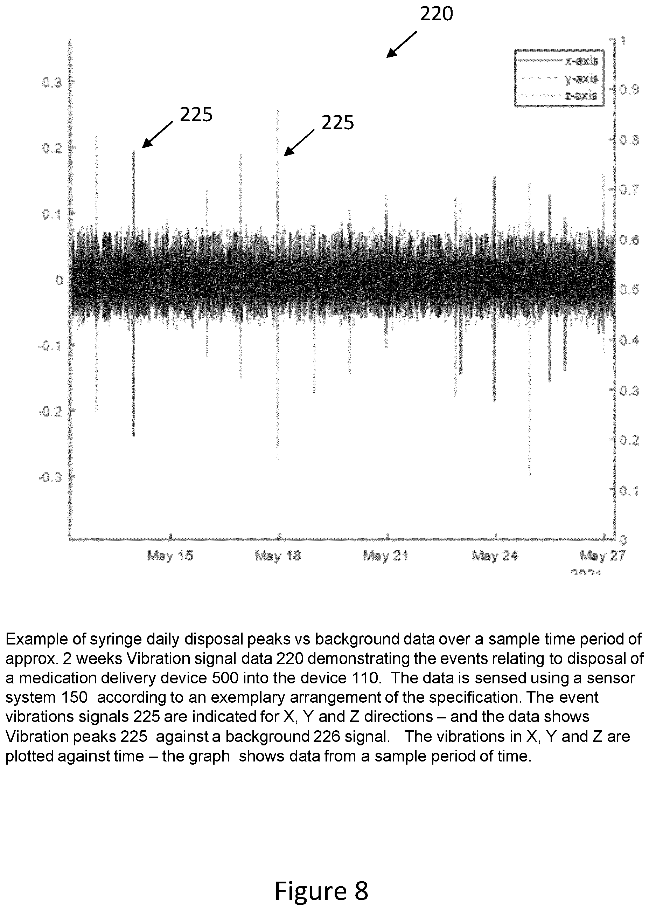

Referring to Figures 7 and 8 some examples of vibration or movement signal data 200 are illustrated. The detected vibrations 220 include those indicative of interaction with the container 110 when a device 500', such as a used medication delivery device 500', is inserted into or deposited into the container 110.

The sensor system 150 may also be configured to detect vibrations 220 indicative or movement of transport of the container 110.

The sensor system 150 may also be configured to detect vibration 220 indicative of movement of the container at the point of care.

The sensor system 150 may also be configured to detect background data i.e. when the container is at rest and between interactions with or movement of the device.

The sensor system is configured to output vibration signal data 220, the signal showing detected vibrations and movement.

Accordingly, the activity, e.g. each interaction or each event at the container 110 as indicated by the detection of vibration signals 220, may include vibration signals arising from deposit events, for example where a user contacts a portion of the container to deposit an item into the container, but where the container itself is not moved and the location is not changed. The activity, interaction or event, indicated by detection of vibration signal 220 may include a movement event at the container for example including where the container is moved, lifted, dropped or other such movement interaction which involves a change in location of the container. The activity could also include the opening and closing (or sliding) of an access panel to a allow the deposit event. The activity or event may also include transport where the container is transported between the end-user/point of care site and the container-supplier/processing-site. By testing and calibration, it is possible to identify different types of interactions and events, including the above listed, from a set of data 220 downloaded from the sensor system.

As noted above the sensor system 150 may operate continuously between consecutive occasions that the container 110 is processed and the sensor system 150 and memory 155 reset. Alternatively, the sensor system 150 may operate continuously once the container is located at the point of care. In a further arrangement, the sensor system 150 may be configured to at particular pre-set times, or when interactions with the container 110 are sensed. The vibration signals 220 detected by the sensor system 150 over a time period are stored locally in the container.

The sensor system 150 is configured to detect movement or vibrations, and may comprise an accelerometer 152 configured to detect x, y and z axis movement and to output a signal 220 indicative of the movement that has been detected.

The act of depositing an item such as a syringe or autoinjector pen into the container creates an event vibration signal 225 (the signal shows a signature of the movement or the vibration) as detected by the sensor. The event vibration signal 225 (detected when a used medication delivery device is inserted into the container) has a particular form depending on the vibrations or movement that occur and are detected. The relationship between a particular interaction at the device e.g. depositing an item and the resulting form of the vibration signal may be determined by calibration of the sensor system 200 in the container 110 during different interactions and activity.

In one arrangement according to the specification, the relationship between particular interactions at the container and the resulting vibration signal data 220 is analysed and processed also with the application of machine learning. The system is configured to analyse the vibration signal data 220 of particular users and that of multiple users. This allows for analysis of how the particular user interacts with the device and how the interactions may change over the time period that the container is at the point of care. This also allows for the machine learning of multiple patient interactions to be applied in the analysis of the received data 200. Clearly the data set for a particular time period for a particular user can be compared also with the data sets for that same user for a previous time period or with different users on a similar care plan. Clearly other analyses of the data may also be performed and provided.

The recorded activity data 200 when analysed is compared with the initial care plan data 210 to allow identification of patterns of use or changes etc.

As discussed above, the recorded contents analysis data 240, 260 is also included in further analyses.

When the stored vibration signal data 220 is reviewed or analysed, occurrences of vibration signals 225 (deposit event) as described above are identifiable within the detected signal. The signal data 220 may accordingly be used to determine when used medication delivery device have been deposited into the container, including the date, time, frequency.

The data 220 provides valuable feedback on the user interaction with the container and relating to how the user is adhering to a prescribed medication schedule when the container was located at the point of care.

In the exemplary arrangement when a sensor system 200 is configured to detect a vibration pattern relating to placing an item in the container, then the system is effectively configured to detect vibration signals relating to multiple interactions involving a user placing an item in the container over a time interval. The sensor system detects vibrations as they occur and outputs the vibration signal 220 which is stored in the memory.

When the detected vibration signals 220 are analysed, the analyser determines whether one or more item deposit events are shown in the signal. With reference to sensor data 220 obtained over a period of time, it is further possible to determine the number of item 500' deposit events, the times, and the frequency of the item deposit events.

Exemplary signals 220, examples of which is shown in Figure 7 and 8, are defined by an x -axis, y -axis and z axis signal, shown against time. The deposit event signal, DES, 225 is as peaks against a normal background signal, NBS 226 indicative of the signal detected by the sensor system 200 when the container 110 is at rest and there is no interaction. The event signals 225 represent occurrence detection of unique time limited events - in this case each being a deposit event.

As illustrated with reference to exemplary Figures 7 and 8 deposit event signal peaks 225 can be differentiated from background or baseline noise in the signal 226. Accordingly, it is possible to determine from the stored data 220 when (date, time and frequency) an item(s) (such as a syringe or autoinjector or other medication delivery device) were deposited into the container 110, by analysis of the data.

The container 110 may be deployed as a container for directly receiving used medication delivery devices. Alternatively, the container may be removably receivable as an inner container in a medication management device 100.

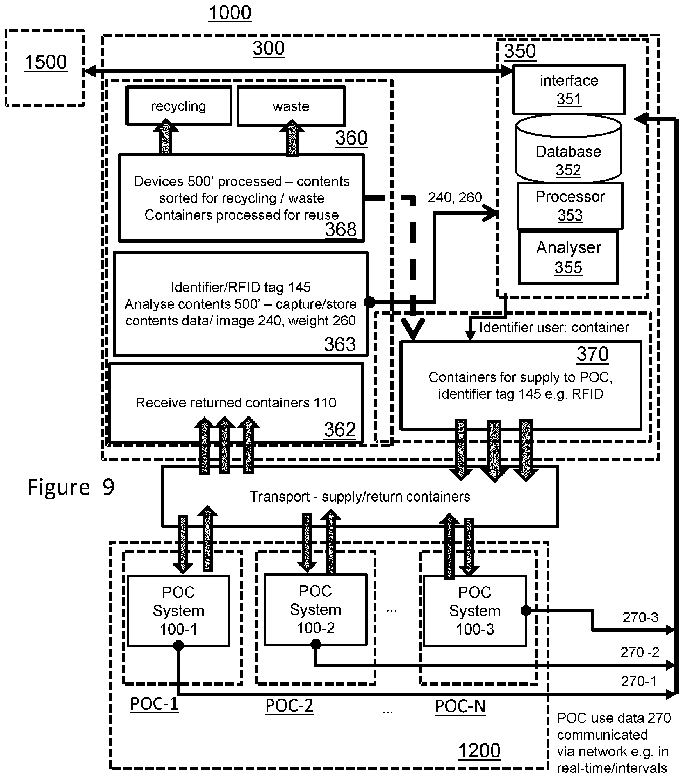

Referring to Figure 9, an alternative arrangement of the system (1000). The arrangement is similar to that of Figs 1 and 2. In the arrangement of Figure 8, a device 100 is provided at the point of care. The device 100 comprises a medication use event detection or recordal means 180 configured to detect or record the occurrence of medication use events. The device 100 further comprises a communication means 185 configured to communicate use event data 270 relating to medication use events at the point of case to the information and data management system 350. Data 270 may be communicated in real time or at intervals.

The device 100 may be configured to contain container 110 for collection of used medication delivery devices 500' at the point of care over a time interval - as described above with reference to the Figures 1 to 8. While the container 110 may comprise sensor system 150 which as described above is configured to detect user interaction and data 220, in the arrangement where the device 100 having medication use event detection or recordal means 180, in the arrangement of Figure 9 the sensor system 150 may be omitted from the container 110.

The other described methods and processing including the processing of returned containers and the capture of image data 240 and weight data 260 pertaining to the contents of the returned container apply to the arrangements of Figure 9 as to the arrangements of Figures 1 and 2.

In the arrangement of Figure 9, the use data 270 may be provided in the place of use data 220 and provides similar feedback - including times/frequency/of medication use events. The data 270 is attributed to the relevant user record - along with the data 240 and 260 - to provide a complete set of data pertaining to use events and contents.

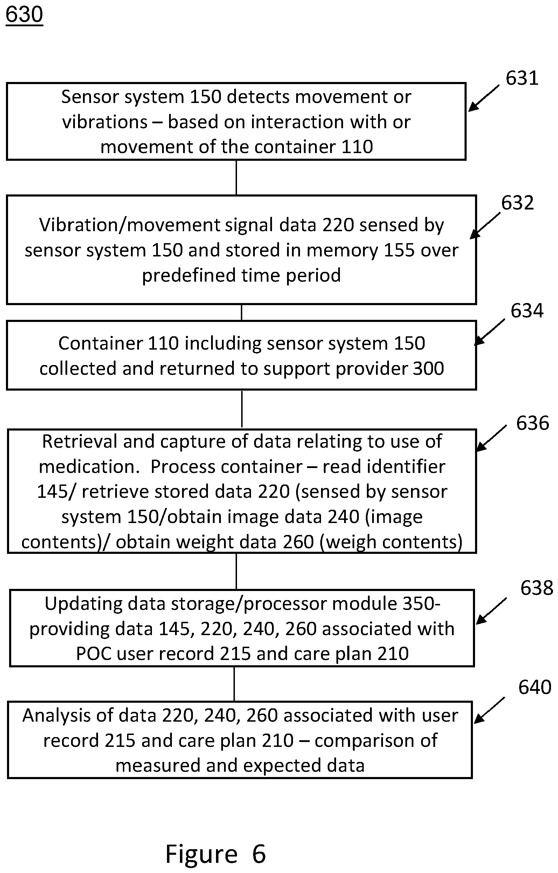

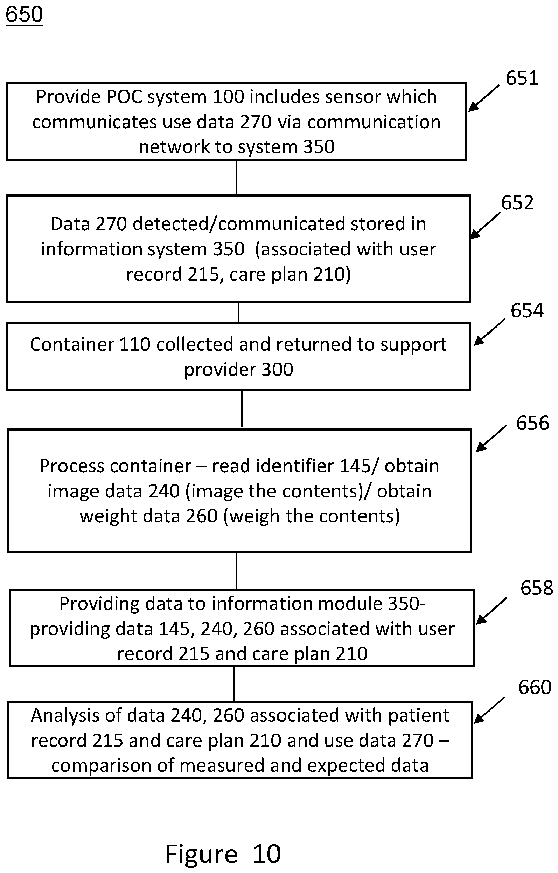

Referring to Figure 10 method 650 - (similar to method 630 of Fig. 6) is described - in this case the use data - as detected by device 100, detector 185 is communicated for example in real-time to the information system 350 by remote communication means. The other steps of the method 650 are similar to those of the method 630.
651 providing POC system 100 including sensor 180 and communication means 185
652 communicating data 270 to system 350. Data 270 detected/communicated stored in information system 350 (associated with user record 215, care plan 210)/
654 Receiving Container 110/contents 500' - container 110 collected and returned to support provider 300
656 Processing container and contents - data 145, 240, 260
658 providing data to information system 350
660 Analysing of data 240, 260 associated with patient record 215 and care plan 210 and use data 270 - comparison of measured and expected data

Further the method of data processing/ analysis/ management provide for analysis of adherence of user to medication plans, identification of any areas of difficulty for users/ provision of support.

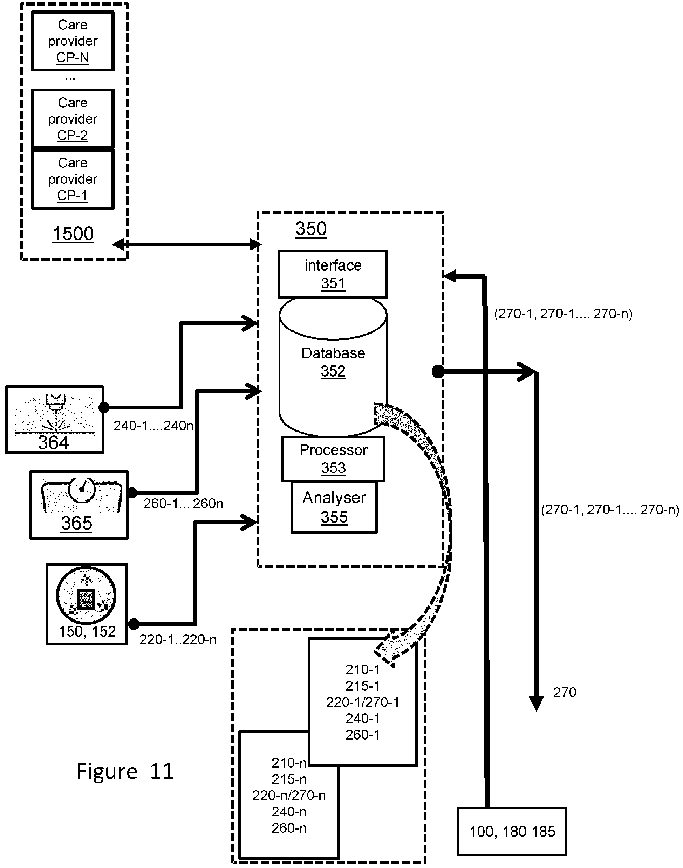

Referring to Figure 11 - an illustration of an overview of an arrangement of the information system 350 and inputs and outputs is provided. The figure also shows an exemplary record 215 and the combinations of data inputs.

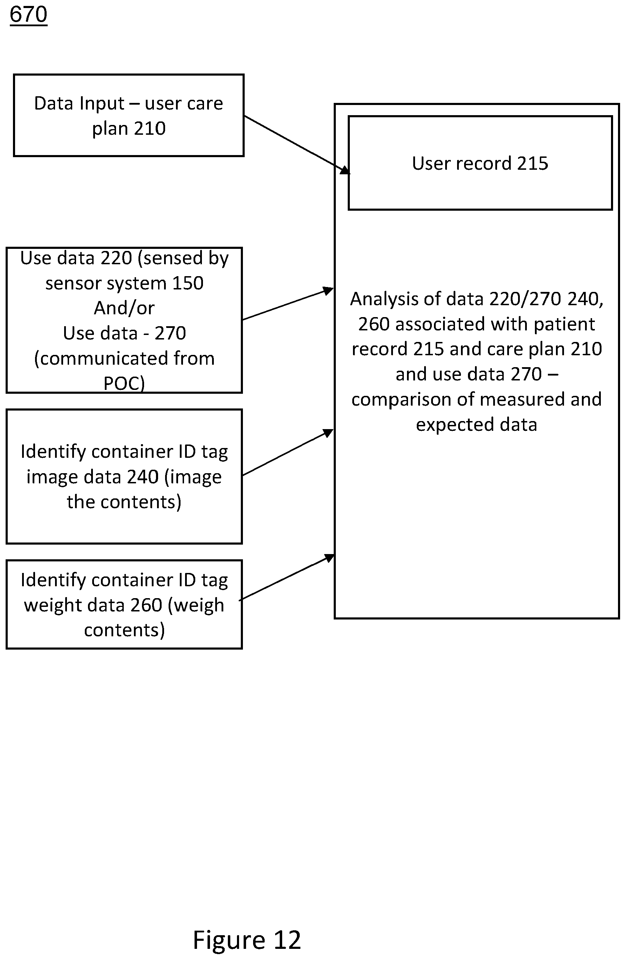

Referring to Figure 12 - the method 670 shows further detail of data inputs to the system 350 and in particular in relation data management of use data and user data.

Referring to Figure 13 - the method 680 shows further detail of data inputs to the system 350 and in particular relating to the container records 290. The container record 290 for a particular container - may include inputs received as the container is passed through various stages of processing. The container record also information a use counter - which includes the number of times that a container has been circulated to an end user point of care. An upper limit on the maximum number of times a container can be recirculated may be set. It is therefore possible to identify when a container has reached the maximum number of re-circulations. Further it is also possible based on logging of processing of the container at various stages to verify that all the required stages of processing have been completed before a container is provided for re-circulation.

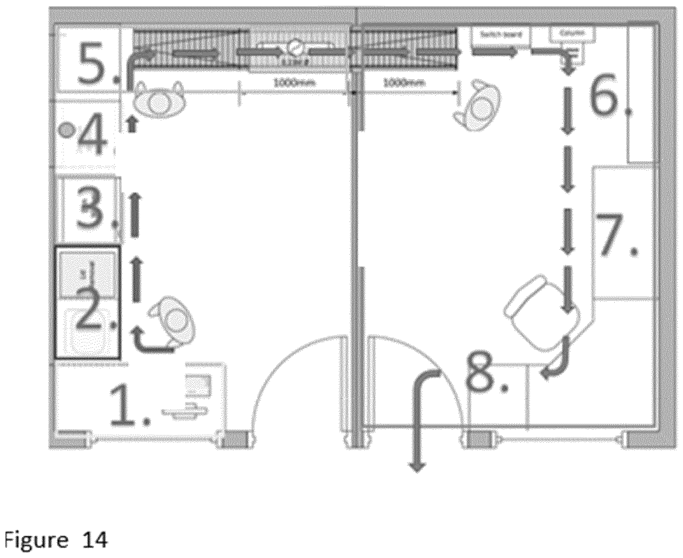

Figure 14 shows an example of an in-line system for the processing of returned containers 110 - based on the methods and systems described above.
**1. Registration:** Containers are scanned within the process room, identified and weighed.
**2. Image Classification:** Lids are removed and the container contents are tipped into a transfer chute.
**3. Sterilisation:** Containers are transferred through a washing station to clean the containers.
**4. Water purification:** wastewater is pumped to enclosed container for treatment.
**5. Relocation:** Containers are transferred from processing room to clean room (container data is collected upon transfer).
**6. Water Evaporation:** containers are moved to air drying location.
**7. Re-registration:** Containers are scanned and relabelled (as required).
**8. Redistribution:** Containers are transferred to for redistribution.

The container may be scanned at one or more of the steps.

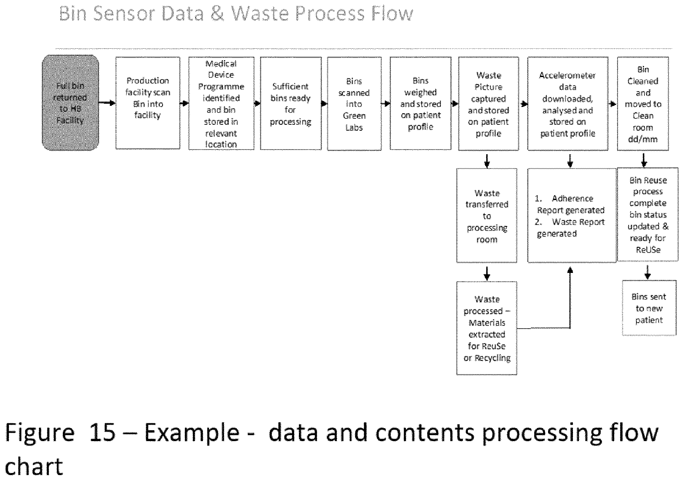

Figure 15 - shows an exemplary - data and container contents 500' processing flow chart according to arrangements of the specification.

Example 1 An exemplary system 1000 illustrates the application of the systems 300 and methods 600 of the present specification in a home point of care POC setting 1200.
1. The patient is prescribed an injection medication and frequency by their Doctor. This forms the medication schedule entered as care plan 210.
2. The patient is provided with a container 110 fitted with a sensor system 150 including an x,y,z sensor 152. The patient's details, including the prescribed medication schedule, are programmed into an interface or dashboard 351 of the system 300 to define the medication programme and schedule of the care plan 210. The details may include the manufacturer or drug brand. The programme may also include a reminder notification schedule. The system 300 may be configured to remind a patient that they are due to take medication at a particular time. The reminder may be via text or SMS to the patient's smart phone as a reminder. The patient at point of care has a supply of medication and medication delivery devices 500.
3. The patient administers their injection and disposes of the used medication delivery device 500' into the sharps container 110.
4. The sensor system 150 detects the interaction event as a vibration signal 225, vibration signal data 220.
5. The patient continues to use the container 110 to dispose of their used medication delivery devices 500'.
6. After 3 months (for example - though it will be appreciated that other time intervals may be selected) a replacement container 110 is sent to the patient and exchanged for the full container.
7. The full container is returned for processing at support provider 300, module 360.
8. The data 220 as detected by the sensor system 150 and stored in the device local memory 155 is downloaded from the sensor system 150.
9. The data 220 is analysed and stored in centralised database 352.
10. The data 220 is available for reporting to the care provider CP-1.
11. Sensor systems 1500 and containers 110 are reused.

The data 220 may be stored in a removable memory 155 at the container 110 which is removed from the container 110 when it is received for processing. Alternatively, the data 220 may be communicated from the memory 155 to the data store 352 at the support provider 300 side.

The data 220 provides information on the user interaction with the container 110 indicative of patterns of use of medication at the point of care and relating to adherence to a medication schedule.

Example 2 The following is an example of how the device, system and methods of the specification would be utilised in a home setting.
1. The patient is prescribed an injection medication and frequency by their Doctor.
2. The patient is provided with a sharps bin fitted with the x,y,z sensor. The patient's details are programmed into the dashboard - manufacturer/drug brand. The programme may also include a reminder notification system which sends an SMS to the patient's smart phone as a reminder.
3. The patient administers their injection and disposes of the used medication into the sharps container.
4. The sensor detects the interaction event.
5. The patient continues to use the bin to dispose of their medication.
6. After 3 months a replacement bin is sent to the patient and exchanged for the full bin.
7. The full bin is returned for processing.
8. The data is downloaded from the sensor.
9. The data is analysed and stored in centralised database.
10. The data can be reported.
11. Sensors and bins are reused.

Advantageously, the container 110 provides a safe and easy to use point of care device. The container 110 may be used as a self-contained and stand-alone system. The sensor system 150 senses and monitors activity at the container and interactions with the container data 220 sensed over a time period the system 110 is deployed at a point of care is stored for later retrieval and analysis. The sensor system 150 may additionally monitor activity at the container as it is transported between the point of care and point of processing. The sensor may additionally monitor activity at the container as it is processed. The container 110 and sensor system 150 are operable without requiring a network connection or power supply. The device 110 is easy for a patient at the point of care to use - there is no requirement for patient to connect the device to a communication network or power and the location of the device is thus not limited by such requirement. The arrangement of the specification accordingly provides a system and method of the monitoring of interaction and activity or transporting of the container 110 off-line without the requirement for real-time monitoring or a real time data communication connection. The system and method therefore advantageously do not require a network power supply or communication connection.

The method and system of the specification advantageously provides support to the overall arrangement for provision of medication to a patient end-user at point of care. This includes feedback of data relating to adherence to a medication schedule and safe and secure collection and disposal/recycling of used medication delivery devices. The system and method are in particular applied for monitoring and detection of user interaction and the management of the container throughout the cycle of deployment of the container between the container supplier and the user. Effectively there is a provided an arrangement for detecting use events and activity relating to the container.

The systems, methods and container 110 comprising a sensor system of the specification have application including for the following:
- Medication Adherence - including supporting and monitoring mediation adherence at POC and supporting care providers;
- Medication use and use medication delivery device disposal habits associated with medication administration;
- Waste management;
- Sharps Exchange Service;
- Patient support services;
- Medical device collection and recycling;
- Waste Management and Circular economy;
   - Patient Support Programmes
   - Data insights in Sharps Exchange Programmes
   - Medication Adherence in Injectable therapies
   - Recycling and waste management programme

The device, system and methods of the specification are applied in particular in the management of injectable medication programmes. In such an application, the container comprises a sharps bin for the safe collection and containing of sharps devices. The used medication delivery devices may comprises one or more of an autoinjector, a syringe, sharps or components including needles.

The container device 110 is configured for use in a medication adherence and management support system 1000. In the overall system 1000 the patient/end-user is provided with a container device 110 and medication 500 at point of care. The system 1000 provides for monitoring of medication adherence for example based on a comparison of deposit events 220 indicative of placement of a used medication delivery device 500' in the container 110. The data relating to placement of used medication delivery device 500' in the container 110 over a period of time can be compared with a user medication schedule of a care plan 210 to provide an indication of whether the time and frequency of placement of medication delivery devices 500 matches with the schedule and to provide an indication or any deviations. The medication adherence and management system 1000 also provides for management of medical waste including used medication delivery devices.

The investment/cost of providing patient support programmes is often extremely high. Patients with chronic conditions, that administer biological therapeutic drugs in the home require significant support in the form of nurse home visits and 24/7 support, training, medication and disease literature, helplines and waste management services. Accordingly, there is a demand for managing costs while improving levels of care for the patient. Managing waste in a more sustainable manner is also a key driver in new programmes and the ability to provide key data metrics around waste generation, end of life and sustainable waste management is becoming more important. Adherence systems that detect in real time have been described, and while providing high levels of support can be expensive to run as they require communication infrastructure to transmit data across networks. While they provide unique patient insights there is still a cost with running connected services. A non-real time system as described herein provides for collection of use data 220 without the need for more expensive smart connected systems. Data 220 is downloaded once the bin comes back for processing. These systems could provide a relatively nominal cost but have the ability to report on important data points including when the patient used the bin to drop their medication, what was the frequency and the interval. From this, a map of their medication administration timeline and habit is determined. Tracking drop off and collection would also be possible as well as collecting key data metrics on waste generation, such as manufacturer/brand, weight, frequency of bin exchange, recycling and reuse metrics, image capture (returned contents) and actual return (return of container and contents) event.

Data reports 295 showing key insights can be provided to care providers that can show important insights on the behaviour of patients taking medications as well as maintaining effective and streamlined Sharps bin/container 110 exchange programmes. Such a system can also provide additional data insights on waste management and recycling programmes providing environmental data figures such as carbon impact via the recycling or reuse activities.

## Claims

1. A point of care device for supporting management of medication at a point of care location the device comprising:
a container having base, top and side walls defining an interior space accessible *via* an opening;
a sensor system comprising:
one or more movement sensors, configured to detect use events which comprise vibrations or movements resulting from interactions with the container, and to output signal data indicative of the movement detected; and
a memory configured to store the detected movement signal data over a defined time period; and
a power supply;
wherein the sensor system is configured for coupling to the container;
the container configurable in a first point of care mode or a second processing mode, wherein in the first point of care mode the container is configured to receive used medication delivery devices into the interior space, and to contain said used medication delivery devices therein, and wherein in the second processing mode the container is configured to allow access to the interior thereof for removal of the used medication delivery devices; and
wherein the sensor system comprises an accelerometer configured to detect vibrations or movement resulting from interactions with the container, in one or more directions (x, y and z axes), and to output a signal indicative of the movement or vibrations that have been detected.

2. The device of claim 1, wherein the sensor system is a self-contained unit and wherein the memory comprises a local memory and the power supply comprises an exchangeable or rechargeable battery.

3. The device of claim 1, wherein the container is a sharps bin or sharps container.

4. The device of claim 1, wherein the used medication delivery devices comprise one or more of: syringes, injector or autoinjector devices, and/or sharps.

5. The device of claim 1, wherein the use events include one or more of deposit events, movement events such as moving, lifting or dropping the container, container location changes, opening and closing of the container opening, or container transport.

6. The device of any preceding claim, wherein the sensor system is configured for operation independently as a stand-alone device, the sensor system having an integrated memory and power supply such that the sensor system does not require real-time connection to an external power supply or to an external communication network or connection to a power supply or external communication network during the time period that the device is located at a point of care site.

7. The device of any preceding claim, wherein the sensor system is removably couplable to the container.

8. The device of any preceding claim, wherein the sensor system is removably insertable into the container, and wherein the sensor system is removable for retrieval of signal data from the memory and/or for connection to a power supply interface for recharging of the power supply.

9. The device of any preceding claim, wherein the container is configured for docking, while the sensor system is in place therein, to a communication interface for retrieval of stored signal data from the memory and/or to a power supply interface for recharging of the power supply.

10. The device of any preceding claim, the container further comprising a receiver for receiving the sensor system and configured to provide secure location of the sensor system relative to the container.

11. The device of any preceding claim, wherein the signal data based on detection of vibrations or movements resulting from end-user interactions with the container to deposit used medication delivery devices into the container provides data indicative of medication use by the end user at the point of care.

12. The device of any preceding claim, wherein the sensor system is configured to detect and store the signal data arising from interactions with the container over a period of time, and wherein the sensor system is configured such that the data is retrievable or downloadable for analysis to allow determination of the dates and times of user interaction with the container, indicative of patterns of use of medication.

13. A point of care medication management system (1000) for supporting and managing point of care medication care plans and for tracking medication care events associated with a point of care medication user, the system comprising:
an information system (350), comprising for each point of care user (POC-1, POC-2) a point of care user record (215-1, 215-2... 2-15-n) including a medication care plan (210-1, 210-2, 210-n) for the point of care user;
a point of care container (110) for collection of used medication devices (500'), wherein the point of care container (100, 110) is deployed to the point of care for the point of care user (POC-1) for a predetermined period of time (900);
a point of care medication use event recordal system (150, 100, 180), configured to record medication use event data (220, 270) at the point of care relating to said period of time that the container is at the point of care, wherein the use event data is provided to the information system (350) and to the point of care user record (215-1);
the system further comprising:
a returned container processing module (360), configured for receiving and processing returned containers (110) and container contents (500'), the container contents comprising the used medication delivery devices collected the point of care for the point of care user (POC-1) over the period of time (900);
wherein the processing of the contents of each returned container comprises:
imaging the contents to obtain image data (240);
weighing the contents to obtain weight data (260);
wherein the image data (240) and weight data (260) pertaining to the contents of the container returned from the point of care user (POC-1) is provided to the information system (350) and to the relevant point of care user record (215-1), and wherein
a point of care device (100) comprising a medication use event detection or recordal means (180) is provided at the point of care (POC-1) and the device is configured to detect or record use event data (270) and to communicate said use event data (270) to the information system (350).

14. The system of claim 13 wherein the container (110) includes an identifier (145) which is read at the processing module (360, 363) to allow the image data (240) and weight data (260) relating to the contents of a container (110) to be attributed to the relevant point of care user record (215-1).

15. The system of claims 13 or 14 wherein the container (110) comprises a sensor system (150) and the sensor system is configured to detect and store use event data (220) and wherein the use event data is retrieved from the returned container (110) at processing.
